# EUROPEAN PATENT SPECIFICATION

(11) **EP 2 313 039 B1**
(45) Date of publication and mention of the grant of the patent: **31.08.2022**
(21) Application number: 09781341.4
(22) Date of filing: 31.07.2009
(51) Int. Cl.: A61F 13/10

(54) **KIT FOR THE TREATMENT OF ONYCHOMYCOSIS**
AUSRÜSTUNG ZUR BEHANDLUNG VON ONYCHOMYKOSE
TROUSSE DE TRAITEMENT D'UNE ONYCHOMYCOSE

(30) Priority: 01.08.2008 FR 0855350
(43) Date of publication of application: 27.04.2011
(73) Proprietor: Pierre Fabre Dermo-Cosmétique, 92100 Boulogne-Billancourt (FR)
(72) Inventor: COUBETERGUES, Héla, F-31240 Saint-jean (FR); VOISARD, Jean-Jacques, F-31380 Montpitol (FR)
(74) Representative: Regimbeau
(86) International application number: PCT/EP2009/059933
(87) International publication number: WO 2010/012824

(56) References cited:
- EP-A- 0 204 230
- EP-A- 1 008 330
- JP-A- 2008 023 111
- US-A- 3 529 597
- US-A- 5 181 914
- US-A- 6 042 845
- US-A1- 2005 166 297
- US-B1- 6 281 239
- US-B1- 6 592 889

## Description

The present invention concerns a flexible occlusive or semi-occlusive dressing.

Onychomycosis is defined as a fungal infection of the nail unit i.e. the matrix or bed of the nail or the nail plate, caused by dermatophytes (of the genus *Trichophyton, Epidermophyton* or *Microsporum),* yeasts *(Candida* or *Malassezia)* or by moulds *(Fusarium).* If it is the hands which are affected, it is most often yeasts which are responsible *(Candida).*

Onychomycosis the most common nail pathology. It concerns 6 to 9% of the general population.

It especially occurs in adults it is rare in children. Its incidence increases with age, and reaches 30% alter the age of 70.

90% of onychomycoses affect the feet and in 9 cases out of 10 the cause is dermatophytes.

The classification of onychomycoses depends on the site of penetration of the infectious agent and its stage of development. A distinction is made between:
- Distal latere sub-ungual onychomycosis (DLSO) is the most frequent form, essentially due to dermatophytes which enter via the side groove and infect the nail bed,
- Proximal ungual onychomycosis (PSO) affecting the nail bed. A rare infection often caused by a dermatophyte.
- White superficial onychomycosis (WSO) due to a dermatophyte or a mould. The fungus enters the nail plate from outside to inside.
- Total dystrophic onychomycosis is the ultimate stage of the preceding forms. It translates as gradual invasion and destruction of the entire nail plate by the fungus.

Onychomycosis never cures spontaneously and is known to be difficult to treat.

Systemic antifungal treatments have highly restrictive side effects.

Three families of topical antifungals can be used to treat onychomycosis: imidazole, morpholine and hydroxypyridone.

Amongst these treatments, film-forming solutions can be found containing either ciclopirox or amorolfine. These are lengthy treatments and must be continued for around one year. Their efficacy is between 10 and 30%.

The difficulty with topical antifungals is the ability to reach the nail bed where the site of the infection lies.

Another approach to topical treatment is to remove the infected part of the nail plate to allow application of an antifungal cream to the nail bed. This removal can be made either chemically (40% urea) or mechanically (filing, forceps).

Chemical removal using urea has selective action on the pathological part of the nail.

Chemical removal using urea has the advantage of being pain-free and of limiting risks of haemorrhage or infection.

US 6,281,239 discloses a kit comprising a urea paste, optionally an antifungal, and occlusive dressings to treat onychomycosis.

EP 0 204 230 discloses a formulation of urea paste containing 0.05 to 1.5 parts by weight of bifonazole and/or clotrimazole, 40 parts by weight of urea, 20 parts by weight of anyhydrous lipophilic wetting agent, 5 parts by weight of white beeswax and 34 parts by weight of white Vaseline for the treatment of onychomycosis.

This paste is marketed by Merck in its Amycor Onychoset^{®} kit in the form of a 10 g tube containing 0.1 g of bifonazole and 4 g of urea with occlusive dressings and a scraper. The treatment consists of softening the nail by means of the keratolytic properties of urea and treating the infection with bifonazole. The ointment is applied once per day and is held in place by an occlusive dressing. Every day the dressing is removed and the softened part of the nail is removed with the scraper after a hot bath. 1 to 3 weeks' treatment are necessary. The treatment must be continued 4 to 8 weeks with the daily application of Amycor0 cream containing 1% bifonazole.

US5,181,914 discloses a care dressing with a T-shape comprising a central part (1) intended to cover the nail, two identical side branches (2) intended to be folded back under the finger on each side of the nail representing the short branches of the T and a tab (3) intended to be folded back longitudinally under the finger positioned in the continuity of the nail before its folding. US5,181,914 does not disclose an occlusive paste-holding dressing with a very low thickness and a structure consisting in a support layer in polymer-based film and an adhesive layer covering the entirety of the support layer.

There is a need for a more efficient, quicker treatment that is less restrictive.

The inventors have evidenced that the critical elements in the treatment and length of treatment of onychomycosis using an urea paste are the occlusive or semi-occlusive capacity of the dressing and the capability of the dressing to prevent leakage of the urea ointment outside the dressing.

They have therefore developed an occlusive or semi-occlusive dressing allowing full occlusion or semi-occlusion to be maintained between the urea ointment and the nail for more than one day, even two days.

The inventors have observed that the nail plate lifts off from the nail bed in less than two weeks and that the nail plate can then be cut away without the need to scrape the softened nail every day.

The subject of the present invention is therefore an occlusive or semi-occlusive thin, flexible dressing as defined in claim 1 whose shape and size are adequate for occluding a fingernail or toenail, and comprising a support layer in polymer-based plastic film coated at least in part with an adhesive layer.

In the meaning of the invention, by « occlusive » is meant the capacity to be impervious to fluids, water vapour and gases.

In the meaning of the invention by « semi-occlusive » is meant the capacity of being impervious to fluids. A semi-occlusive dressing is impervious to fluids such as water but is permeable to gases e.g. air.

In the meaning of the present invention by « occlusion » is meant the capacity of the dressing to create a enclosed space around the nail.

The dressing of the invention is of upturned T-shape, comprising a central part (1) intended to cover the nail, 2 identical side branches (2) intended to be folded back under the finger either side of the nail, representing the short branches of the T and a tab (3) intended to be folded back longitudinally under the finger positioned in the continuity of the nail before being folded and representing the long branch of the T.

In the meaning of the present invention « finger » is used indifferently to mean the finger of the toe of a human being.

According to this embodiment the side branches (2) extend perpendicular to the tab (3).

Preferably the dressing of the invention and the tab (3) have an axis of symmetry. Further preferably, these two axes of symmetry are identical.

Optionally, the central part (1), opposite the tab (3), may have a tab (4) which is not intended to be folded back but is intended to improve the comfort at the first phalanx after the nail. Preferably, the length of the additional tab (4) is less than 10 mm.

The support film is a polymer-based plastic film, said polymer being chosen from among the group consisting of polyolefins such as polyethylene, copolymers of polypropylene, homopolypropylene, polybutene, and polymethylpentene, ethylenevinyl acetate copolymers, ionomer resin, ethylene-methacrylic acid copolymers, ethylene-methacrylate ester copolymers, ethylene-butyne copolymers, ethylene-hexene copolymers, polyurethane, polyesters such as polyethylene terephthalate, polyimide, polyethercetone, polystyrene, polyvinyl chloride, polyvinylidene chloride, fluororesin, silicone resin, cellulose resin.

Preferably the film used is a thin film in transparent or translucent flexible material such as a film containing polyurethane or made solely of polyurethane.

The thickness of the dressing of the invention is less than 60 µm, and further preferably less than 50 µm, and in particularly preferred manner it is 40 µm or less. This thickness does not include any optional protection of the adhesive layer before use.

Preferably, the thickness of the dressing of the invention is more than 10 µm, further preferably more than 20 µm. This thickness does not include any optional protection of the adhesive layer before use.

The adhesive layer fully covers the support layer.

The adhesive used is not particularly limited provided it gives rise to little or no skin irritation. The adhesive is chosen from among known materials, notably hypoallergenic adhesives containing acrylic polymers or copolymers. Examples of suitable adhesives comprise polyurethane adhesives, silicone adhesives and acrylic adhesives. Medical grade acrylic adhesives are particularly suitable.

The quantity of adhesive lies between 25 and 80 g per square metre of support layer, e.g. 30 to 60 g/m2, for example 30 to 50 g/m2, and preferably it is 40 g/m2.

The peel adhesion strength of the adhesive layer of the dressing according to the invention is greater than 10.0N/25mm (for an angle 180°, peel rate of 300 ± 30 mm/min, temperature of 23 ± 2°C), and in particularly preferred manner it is 12.0N/25mm (for an angle of 180°, peel rate of 300 ± 30 mm/min, temperature of 23 ± 2°C). Peel adhesion of N/25 mm is measured using the following method, corresponding to standard AFERA 4001 for measurement of the adhering performance of an adhesive: a strip of adhesive is applied to a standard metal plate, preferably in stainless steel, which is then attached vertically in the moving clamp of a traction testing machine. The clamp pulls the free end of the sample at an angle of 180° relative to the plate. Peel adhesion strength corresponds to the force required to peel 25 mm of the adhesive strip from the plate, continuously, the separating line between the plate and the strip forming right angles with the direction of applied force, at a speed of 300 ± 30 mm/min.

Peel adhesion is correlated with the quantity of adhesive per square metre of support layer. It is around 8.0N/25mm per 25 g/m2 of adhesive. It is around 12.0N/25mm per 40 g/m2 of adhesive. According to the present invention, peel adhesion is at least equal to 10.0N/25mm, and preferably it is 12.0N/25mm.

Preferably, the peel adhesion strength of the adhesive layer is the result of the mean peel adhesion strengths of three samples of the adhesive layer.

Preferably the length of the dressing of the invention, length being defined as the sum of the width of part (1) and length of part (3) and optionally length of part (4) lies between 6 and 8 cm, more preferably between 65 and 70 mm and further preferably it is 70 mm ± 2 mm.

Preferably the width of the dressing of the invention, width being defined as the sum of the length of part (1) and length of parts (2) lies between 7.5 and 9.5 cm, more preferably between 80 and 90 mm, further preferably it is 84 mm ± 2 mm.

The central part (1) is approximately contained within a rectangle. This rectangle has the following dimensions: between 35 and 55 mm in length, preferably 45 mm in length, and between 30 and 50 mm in width, preferably 40 mm.

The length of the tab (3) is greater than the length of the side branches (2).

The length of the tab (3) lies between 20 and 40 mm, and is preferably 30 mm.

The length of the side branches (2) lies between 15 et 25 mm, and is preferably 20 mm.

Preferably the dressing of the invention is transparent or translucent.

The adhesive layer covers the entirety of the support layer of the dressing according to the invention.

Preferably the dressing of the invention has an anti-adhering protector over the free adhesive layer (not covered by the support layer) which is removed before use. As an example, the protector is made of silicon paper or plastic material such as polyethylene or polyester treated to be non-adhering.

Preferably the protector consists of 2, 3 or 4 parts.

In particularly preferred manner, the protector consists of 4 parts, one part at least covering the central part (1), one part at least covering part of the tab (3) and two parts covering at least part each side branch (2).

Preferably the dressing of the invention has no cushion pad. Usually, dressings comprise a cushion pad generally in woven or non-woven fibres to absorb body fluids seeping from the wound to be protected. The presence of a cushion pad is unfavourable for the dressing of the invention which is not intended to protect a wound but to hold a urea paste in place. Any cushion pad would crush the urea paste and promote its leakage outside the dressing and/or would absorb the urea paste from the surface to be treated.

The dressing of the invention consists of two layers:
- a support layer, preferably in polyurethane,
- an adhesive layer covering the entirety of the support layer.

Optionally, the adhesive layer of the dressing according to the invention is covered, before use of the dressing, with a protector preferably in silicone paper and preferably consisting of 4 parts, one part at least partly covering the central part (1), one part at least partly covering the tab (3) and two parts at least partly covering each side branch (2).

The manufacture of the dressing according to the invention has recourse to techniques known to persons skilled in the art. The support layer is coated with adhesive and the adhesive is covered by the protector.

One method of application of the dressing according to the invention is as follows:
(a) remove the paper covering the adhesive part,
(b) apply the central part of the dressing onto the nail centring the nail relative to the central part,
(c) apply the tab (3) then the side branches (2) under the finger.

Another method to apply the dressing of the invention is the following:
(a) remove the paper (10) covering the adhesive part of the tab (3)
(b) apply the pad of the finger to be treated onto the dressing,
(c) apply the urea paste to the nail,
(d) remove the adhesive part from the remainder of the dressing,
(e) fold the tab (3) over the nail,
(f) secure the tab (3) onto the top of finger then the side branches (2) underneath the finger, and optionally
(g) reposition the urea paste over the nail by exerting pressure.

The dressings according to the invention are individually packed in a sealed envelope or several dressings per envelope. The medium inside the envelope may or may not be sterile. Preferably, the dressings of the invention are not sterile packaged.

A further subject of the present invention concerns a kit comprising:
- a container of urea paste,
- occlusive or semi-occlusive dressings according to the invention.

The dressing of the invention is particularly suitable for use in the treatment of onychomycosis by applying an urea paste to the affected nail. The dressing of the invention holds the paste in place on the nail and increases the keratolytic properties of the urea via its occlusive or semi-occlusive property. Additionally, it can be held in place for a longer time than prior art occlusive dressings e.g. up to two days despite the very greasy urea paste applied to the nail and despite utilization on toenails which undergo mechanical stresses through the wearing of shoes. Optionally, the transparency of the dressing allows proper visibility of the positioning of the dressing on the nail to be treated relative to the quantity of urea paste which has been applied, and it is therefore possible to best adjust positioning of the dressing on the skin to ensure good adhesion thereto. The flexibility of the dressing allows good positioning of the adhesive part onto the skin without crushing the urea paste either side of the nail. The urea paste of the kit according to the invention contains around 20 to 50 % urea, preferably 30 to 45 %, further preferably 40 % urea.

Additionally, the urea paste in the kit of the invention comprises lanoline and white vaseline. Preferably the urea paste in the kit of the invention comprises 10 to 30 % lanoline, more preferably 20 % lanoline. Preferably the urea paste in the kit of the invention comprises 30 to 50 % white vaseline, more preferably 40 % white vaseline.

Optionally, the urea paste of the invention also contains an aluminium and magnesium silicate and/or hydrophilic silica. According to this embodiment, the urea paste of the invention preferably contains 0.1 % to 1 % aluminium and magnesium silicate, particularly preferably 0.4 %. According to this embodiment, the urea paste in the kit of the invention contains between 0.05 % and 0.5 % hydrophilic silica, particularly preferably 0.4 % if hydrophilic silica is added alone and 0.06 % if a mixture of hydrophilic silica and aluminium and magnesium silicate is added. In this embodiment, the urea paste in the kit of the invention preferably contains around 20% lanoline, around 49.6% white vaseline and around 0.4 % silica or a mixture of silica and aluminium and magnesium silicate.

According to one embodiment of the invention, the urea paste consists of around 20% lanoline, around 40% white vaseline and around 40% of a urea/magnesium silicate/hydrophilic silica mixture. Preferably the weight ratio of the constituents of the urea/magnesium silicate/hydrophilic silica mixture is around 98.85 :1 :0.15 respectively.

Preferably, the white vaseline in the urea paste of the kit according to the invention is Syntadex A Codex white vaseline.

Preferably, the hydrophilic silica of the urea paste in the kit of the invention is Aerosil V200 or Aerosil R972.

The container for the urea paste in the kit of the invention may be ajar, a tube, a bottle. Preferably it is a tube which facilitates application of the urea paste to the nail.

The container for the urea paste in the kit according to the invention may contain 5 to 50 g urea paste, preferably 10 to 30 g and further preferably around 10 g. The kit according to the invention preferably contains 10 to 50 dressings.

The kit of the invention preferably contains a number of dressings that is a multiple of 7.

The kit of the invention preferably contains 7, 14, 21 or 28 dressings, particularly preferably 21 dressings i.e. a quantity designed for three weeks' treatment.

Other characteristics and advantages of the invention will become better apparent on reading the description of one embodiment of the invention. The description refers to the appended figures in which:
Figure 1 is a plan view of a dressing according to the invention.
Figures 2 and 3 illustrate the method of applying the dressing in Figure 1 to a fingernail.

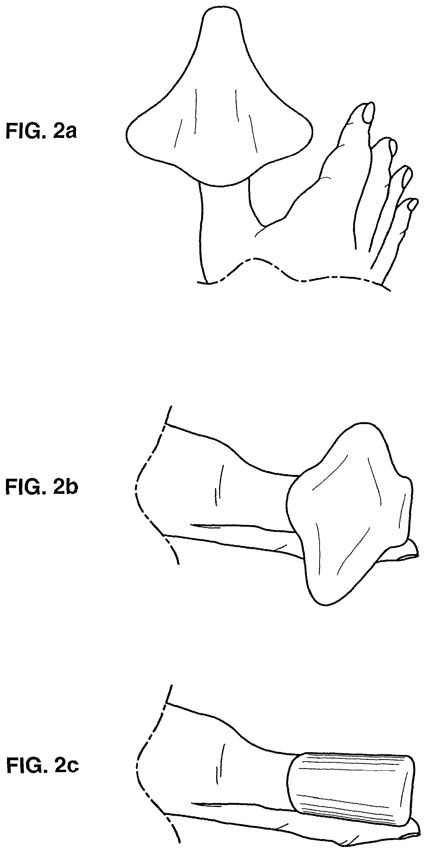

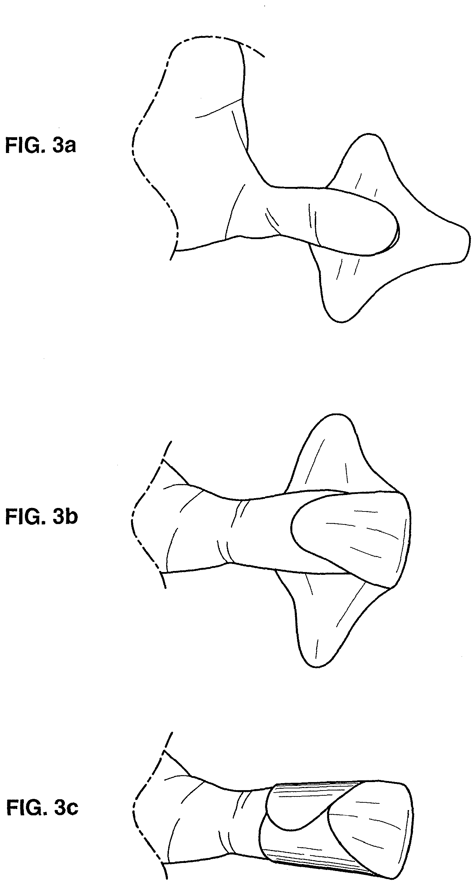

Figure 2 is a view from the side of the nail, whereas Figure 3 is a view on the side of the finger pad.

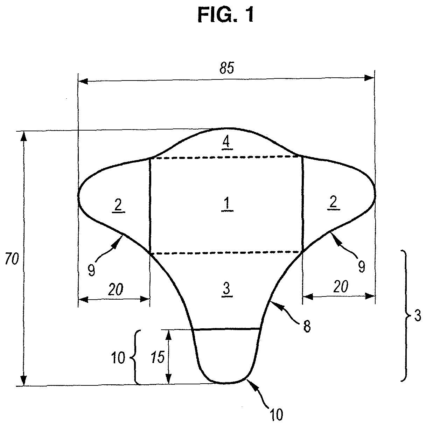

The dotted lines in Figure 1 are solely figurative to separate the different parts 1, 2, 3 and 4 but do not correspond to any material element. The solid lines correspond to the limits of the protector parts.

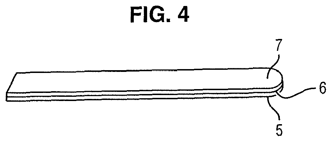

Figure 4 is a longitudinal section view of Figure 1.

The dressing of the invention will be explained with reference to Figures 1 to 4.

As shown in Figure 1, the dressing of the invention is of approximate upturned T shape and comprises a central part (1) of rectangular shape, 2 side branches (2) on the small sides of the rectangle, a tab (3) on one of the long sides of the rectangle and a short tab (4) on the long side of the rectangle opposite the side with tab (3). The solid lines represent the limits of 3 out of the 4 parts forming the protector. The dotted lines determine parts (1) to (4) for the sole purpose of defining the invention, except the upper dotted line which corresponds not only to the limit between part (1) and tab (4) but also to a limit of a protector element. The lower dotted line corresponds to the limit between part (1) and tab (3) solely for the purpose of defining the invention, it does not correspond to any material element.

The dressing of the invention has a layer of polymer-based plastic film (5), an adhesive layer (6) covering the entirety of the polymer-based plastic film and a silicon paper (7) covering the adhesive part to be removed before use (Figure 4).

As shown in Figures 2a and 3a, once the silicone paper part (8) covering part (1) has been removed, the nail is centred on the central part (1) on the adhesive side and the central part (1) is pressed to adhere to the nail.

The tab (3) then lies in the continuity of the nail at the end of the finger and the side branches lie on each longitudinal side of the nail.

The silicone paper part (8) is then removed and the part (10) partly covering the tab (3) is removed. Next, as shown Figures 2b and 3b, the tab is folded longitudinally underneath the finger and adheres to the finger pad i.e. that part of the end of the finger opposite the nail.

Finally, the parts of silicon paper (9) covering the side branches (2) are removed. As shown Figures 2c and 3c, the side branches are then folded over and adhere to the tab (3).

The following examples illustrate the invention without the limiting the scope thereof.

### Example 1: urea caste

Composition: 40% urea, 20% lanoline and 40% Syntadex A white vaseline.

### Example 2 : Kit of the invention and its use

One example of a kit according to the invention consists of a cardboard box containing 21 dressings according to the invention and a 10 g tube of urea paste according to Example 1 i.e. the quantities needed for daily treatment over three weeks.

The affected nail is coated with the urea paste using the tube in the kit and a dressing from the kit is applied. This application is renewed every 24 hours.

When the nail plate has been softened and become detached from the bed of the nail, it is cut off using scissors.

### Example 3: urea paste

Preparation of a Urea / Neusilin / Aerosil V200 mixture consisting of:

| | |
|---|---|
| - Urea | 98.85% (w/w) |
| - Neusilin^{®} (magnesium aluminometasilicate) | 1% (w/w) |
| - Aerosil^{®} V200 (hydrophilic silica) | 0.15% (w/w) |
| Micronized Urea/Neusilin^{®}!AerOsil^{®} V200 mixture | 40.465 (40% urea) |
| Lanoline | |
| White vaseline | 20% |
| | 39.535% |

### Example 4: Protocol for clinical trial

Primary objective: to evaluate the efficacy of the ointment in Example 1 versus an ointment associating bifonazole and urea on complete removal of the region of the nail plate clinically affected by onychomycosis of the toenails.

One of the secondary objectives: to evaluate tolerance to the ointment in Example 1.

### General methodology:

Controlled, randomized, multicentre open trial versus reference treatment in parallel groups.

**Trial product:** Ointment in Example 1

**Route of administration:** Topical

**Mode administered:** Once a day, the patient applief the necessary quantity of ointment to fully cover the surface of the infected nail, covered by an occlusive dressing for 24 hours. The application was renewed and the dressing changed every day after removing excess ointment remaining after the previous application.

**Length of treatment:** 3 weeks, or less if complete removal of the region of the clinically infected target nail plate was obtained earlier. In this case, the patient returned as soon as possible for ad hoc consultation considered as the last consultation of period 2, and treatment was stopped.

### Evaluation criteria:

### Chief criterion for efficacy

Complete removal of the region of the infected target nail plate after 3 weeks' treatment, evaluated under a blind study by a committee of independent specialists on the basis of standardized photographs taken on D21 compared with those taken on DO using the following two-point scale: 0 = Failure; 1 = Success

### Some examples of secondary criteria of efficacy

Complete removal of the region of the clinically infected target nail plate after 3 weeks' treatment, evaluated by the investigator on the basis of clinical examination on D21 compared with photographs taken on DO using the following two-point scale : 0 = Failure; 1 = Success.

Patient assessment on D21 of ease of use of the treatment on the following 4-point scale: 0 = Not at all satisfactory; 1 = Little satisfactory; 2 = Satisfactory; 3 = Very satisfactory .

## Claims

1. Flexible occlusive or semi-occlusive dressing having a thickness of less than 60 µm, and of adequate shape and size for occlusion of a fingernail or toenail and consisting of a support layer in polymer-based plastic film and an adhesive layer covering the entirety of the support layer, **characterized by** an up-turned T-shape comprising a central part (1) intended to cover the nail, two identical side branches (2) intended to be folded back under the finger on each side of the nail representing the short branches of the T and a tab (3) intended to be folded back longitudinally under the finger positioned in the continuity of the nail before its folding and representing the long branch of the T and **characterized by** a peel adhesion strength of the adhesive layer being greater than 10.0N/25mm measured using standard AFERA 4001 (for an angle of 180°, peel rate of 300 ± 30 mm/min, temperature of 23 ± 2°C) .

2. Dressing according to claim 1 having an additional tab (4) of length shorter than 10 mm opposite the tab (3).

3. Dressing according to any of the preceding claims, the support layer being a polyurethane-based plastic film.

4. Kit comprising:
- A flexible occlusive or semi-occlusive dressing as defined in one of claims 1 to 3,
- a container of urea paste.

5. Kit according to claim 4, the urea paste containing around 40 % urea.

6. Kit according to any of claims 4 to 5, the urea paste containing around 20 % lanoline and around 40 % white vaseline.

## Patentansprüche

1. Flexibler Okklusiv- oder Semiokklusivverband, der eine Dicke von weniger als 60 µm und einer adäquaten Form und Größe zur Okklusion eines Fingernagels oder Zehennagels aufweist und aus einer Trägerschicht in einer Kunststofffolie auf Polymer-Basis und einer Klebeschicht, die die Gesamtheit der Trägerschicht abdeckt, besteht, **gekennzeichnet durch** eine umgedrehten T-Form, umfassend einen zentralen Teil (1), der dazu vorgesehen ist, den Nagel abzudecken, zwei identische Seitenarme (2), die dazu vorgesehen sind, unter den Finger auf jeder Seite des Nagels zurückgeklappt zu werden, die kurzen Arme des T's darstellend, und eine Lasche (3), die dazu vorgesehen ist, längs unter den Finger zurückgeklappt zu werden, vor ihrem Klappen in der Fortführung des Nagels positioniert und den langen Arm des T's darstellend, und **gekennzeichnet durch** eine Schälfestigkeit der Klebeschicht von mehr als 10,0 N/25 mm, unter dem Standard AFERA 4001 (für einen Winkel von 180°, Schälrate von 300 ± 30 mm/min, Temperatur von 23 ± 2 °C) gemessen.

2. Verband nach Anspruch 1, der eine zusätzliche Lasche (4) mit einer Länge, die kürzer als 10 mm ist, entgegengesetzt zu der Lasche (3) aufweist.

3. Verband nach einem der vorhergehenden Ansprüche, wobei die Trägerschicht eine Kunststofffolie auf Polyurethan-Basis ist.

4. Kit, umfassend:
- einen flexiblen Okklusiv- oder Semiokklusivverband, wie in einem der Ansprüche 1 bis 3 definiert,
- einen Behälter mit Harnstoffpaste.

5. Kit nach Anspruch 4, wobei die Harnstoffpaste etwa 40 % Harnstoff enthält.

6. Kit nach einem der Ansprüche 4 bis 5, wobei die Harnstoffpaste etwa 20 % Lanolin und etwa 40 % weiße Vaseline enthält.

## Revendications

1. Pansement flexible occlusif ou semi-occlusif ayant une épaisseur inférieure à 60 µm, et ayant une forme et une taille adéquates pour l'occlusion d'un ongle de doigt ou d'un ongle d'orteil et consistant en une couche de support en un film plastique à base de polymère et une couche adhésive couvrant la totalité de la couche de support, **caractérisé par** une forme de T retourné vers le haut comprenant une partie centrale (1) destinée à couvrir l'ongle, deux branches latérales identiques (2) destinées à être repliées sous le doigt sur chaque côté de l'ongle représentant les branches courtes du T, et une languette (3) destinée à être repliée longitudinalement sous le doigt, positionnée dans la continuité de l'ongle avant son repliement et représentant la branche longue du T, et **caractérisé par** une force d'adhérence au pelage de la couche adhésive supérieure à 10,0 N/25 mm, mesurée conformément à la norme AFERA 4001 (pour un angle de 180°C, une vitesse de pelage de 300 ± 30 mm/min, une température de 23 ± 2°C).

2. Pansement selon la revendication 1, ayant une languette additionnelle (4) de longueur inférieure à 10 mm opposée à la languette (3).

3. Pansement selon l'une quelconque des revendications précédentes, dans lequel la couche de support est un film plastique à base de polyuréthanne.

4. Trousse comprenant :
- un pansement flexible occlusif ou semi-occlusif tel que défini dans l'une quelconque des revendications 1 à 3,
- un récipient de pâte d'urée.

5. Trousse selon la revendication 4, dans laquelle la pâte d'urée contient environ 40 % d'urée.

6. Trousse selon la revendication 4 ou 5, dans laquelle la pâte d'urée contient environ 20 % de lanoline et environ 40 % de vaseline blanche.
